Europäisches Patentamt

European Patent Office

Office européen des brevets

(11) Veröffentlichungsnummer : **0 036 130**
**B1**

(19)

(12) **EUROPÄISCHE PATENTSCHRIFT**

(45) Veröffentlichungstag der Patentschrift :
15.12.82

(51) Int. Cl.³ : **C 07 C 15/073**, C 07 C 2/68,
C 07 C 7/148

(21) Anmeldenummer : **81101539.5**

(22) Anmeldetag : **04.03.81**

(54) **Verfahren zur Umsetzung des bei der Synthese von Ethylbenzol erhaltenen Destillationsrückstandes.**

(30) Priorität : **13.03.80 DE 3009632**

(43) Veröffentlichungstag der Anmeldung :
**23.09.81 (Patentblatt 81/38)**

(45) Bekanntmachung des Hinweises auf die Patenterteilung : **15.12.82 Patentblatt 82/50**

(84) Benannte Vertragsstaaten :
**BE DE FR NL**

(56) Entgegenhaltungen :
**US A 2 498 567**
**US A 3 843 739**
**US A 4 179 473**

(73) Patentinhaber : **BASF Aktiengesellschaft .**
**Carl-Bosch-Strasse 38**
**D-6700 Ludwigshafen (DE)**

(72) Erfinder : **Heimlich, Guenther, Dr.**
**Nr. 56 1/2**
**D-8229 Ainring (DE)**
Erfinder : **Tremmel, Gregor**
**Zeppelinstrasse 12**
**D-6718 Gruenstadt (DE)**
Erfinder : **Lieb, Manfred**
**Radestrasse 5**
**D-6700 Ludwigshafen (DE)**
Erfinder : **Lausberg, Dietrich, Dr.**
**Merziger Strasse 21**
**D-6700 Ludwigshafen (DE)**

Verfahren zur Umsetzung des bei der Synthese von Ethylbenzol erhaltenen Destillationsrückstandes

Die Erfindung betrifft ein Verfahren zur Umsetzung des bei der Synthese von Ethylbenzol durch Alkylieren von Benzol mit Ethylen in Gegenwart von Katalysatoren erhaltenen und anschließend abgetrennten Destillationsrückstandes.

Die Umsetzung von Benzol mit Ethylen zu Ethylbenzol in Gegenwart von Friedel Crafts-Katalysatoren ist ein technisch im großen Umfang ausgeübtes Verfahren. Einzelheiten verschiedener Verfahren sind in den folgenden Druckschriften beschrieben :

(I) Kunststoff-Handbuch, Bd. V, Polystyrol, Carl Hanser-Verlag (1969), Seiten 18 bis 23.
(II) DE-AS 19 07 011
(III) DE-AS 24 45 797 und
(IV) US-PS 3 751 504.

Die Herstellung von Ethylbenzol durch Alkylierung von Benzol mit Ethylen in Gegenwart von Friedel-Crafts-Katalysatoren und Promotoren hierfür, ergibt in der Regel ein Reaktionsgemisch, das außer dem Wertprodukt Ethylbenzol nicht umgesetztes Benzol, sowie Diethylbenzol, Triethylbenzol und außerdem hochsiedende Polyethylbenzole und kondensierte Aromaten, zum Teil unbekannter Struktur, enthält.

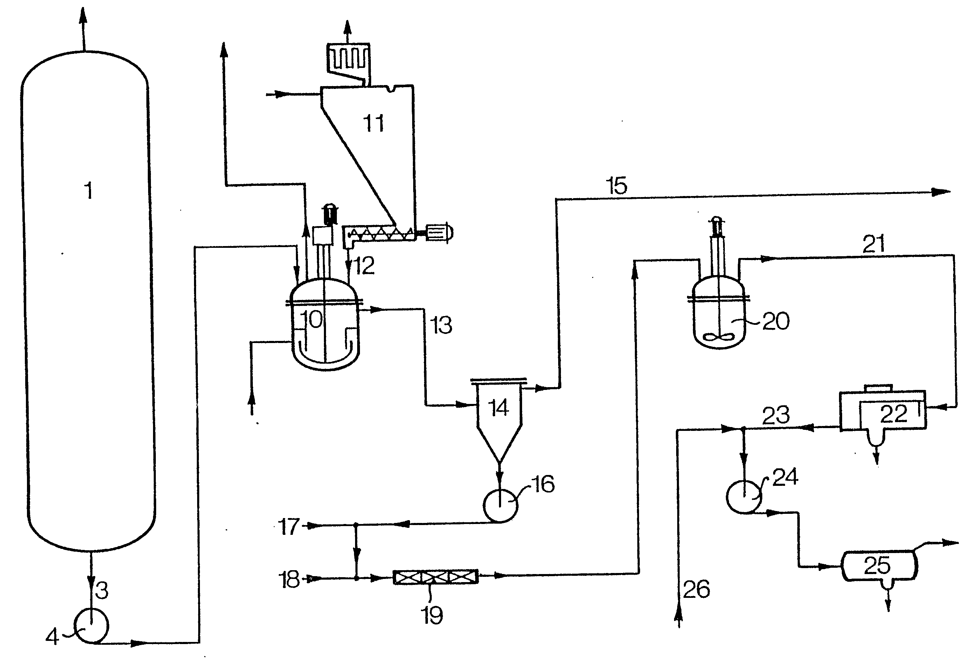

Unabhängig vom Verfahren zur Herstellung des Ethylbenzols muß das Wertprodukt, das Ethylbenzol, von dem nichtumgesetzten Benzol und den Nebenprodukten abgetrennt werden. Die Aufarbeitung des Reaktionsgemisches erfolgt durch Fraktionierung in verschiedenen, kontinuierlich betriebenen, Kolonnen, von denen die wichtigsten als Benzolkolonne, Ethylbenzolkolonne bzw. Polyethylbenzolkolonne (PEB-Kolonne) bezeichnet werden. In der letztgenannten Kolonne wird als Kopfprodukt eine Mischung aus überwiegend, ca. 70 Gew.-%, Diethylbenzolen, sowie geringen Mengen an höheralkylierten Benzolen abgezogen, vgl. insbesondere (1), Seite 24, Bild 7.

Das Sumpfprodukt der Polyäthylbenzolkolonne stellt eine Benzol- und Ethylbenzol-freie Mischung dar, die geringe Mengen an Diethylbenzol, Triethylbenzol sowie einen etwas größeren Anteil an noch höheren Polyethylbenzolen, und überwiegend kondensierte Aromaten aufweist. Dieses Sumpfprodukt wird gelegentlich auch als Fließöl oder Teer bezeichnet. Bei den bekannten Verfahren wird dieses Sumpfprodukt in der Regel verworfen oder zur Aufheizung eines Wärmeträgeröls (Diphenylanlage) verbrannt [vgl. dazu (III), Spalte 8, Zeile 31 ff]. Auf diese Weise wird der Destillationsrückstand aus dem Kreislauf der Ethylbenzolsynthese entzogen ; die Ausbeute an Ethylbenzol wird dadurch in den bekannten Verfahren verringert.

Es bestand nunmehr die Aufgabe, die Ausbeute der Ethylbenzol-Verfahrens durch Umwandlung des Destillationsrückstandes in verwertbare Produkte zu erhöhen. In (I) [vgl. Seite 25, 1. Absatz] ist angegeben, daß der Destillationsrückstand nicht rückalkyliert werden kann. Ganz offensichtlich wird die Rückalkylierung, d.h. die Umsetzung des Rückstandes mit Benzol in Gegenwart von Katalysatoren als die einzige Möglichkeit für die chemische Aufarbeitung dieses Rückstandes angesehen. Es mußte daher überraschen, daß es gelingt, in Abwesenheit von Benzol durch eine chemische Behandlung des Destillationsrückstandes bei erhöhten Temperaturen mit Aluminiumchlorid und Chlorwasserstoff einen Anteil von mehr als 50 % des Rückstandes in chemisch verwertbare Produkte zu überführen.

Die Erfindung betrifft daher ein Verfahren zur Umsetzung des bei der Synthese von Ethylbenzol durch Alkylieren von Benzol mit Ethylen in Gegenwart von Katalysatoren erhaltenen und anschließend abgetrennten Destillationsrückstandes, wobei man den Destillationsrückstand in Abwesenheit von Benzol in Gegenwart von 2 bis 20 Gew.-% wasserfreiem $AlCl_3$, bezogen auf den Rückstand und in Gegenwart von etwa äquimolaren Mengen Chlorwasserstoff, bezogen auf $AlCl_3$, bei Temperaturen von 30 bis 150 °C und bei Verweilzeiten von 20 Minuten bis zu 6 Stunden, umsetzt, und das Reaktionsprodukt dieser Umsetzung in eine

a) spezifisch leichtere organische Phase, die im wesentlichen chemisch verwertbare Wertstoffe enthält, und

b) eine spezifisch schwerere Phase, die im wesentlichen hochsiedende Aromaten und Katalysatorrückstände enthält, trennt und aus der spezifisch leichteren Phase a) die Wertstoffe in üblicher Weise durch Destillation gewinnt.

Zur Durchführung des erfindungsgemäßen Verfahren werden bevorzugt die bei der Synthese von Ethylbenzol durch Alkylieren von Benzol mit Ethylen in Gegenwart von Friedel-Crafts-Katalysatoren, wie wasserfreiem Aluminiumchlorid und von Chlorwasserstoff oder Ethylchlorid als Promotor anfallenden Rückstände verwendet. Die Destillationsrückstände können jedoch auch aus dem unter Verwendung von Zeolithkatalysatoren nach dem Dampfphasenverfahren hergestellten Ethylbenzol stammen. Der Destillationsrückstand kann je nach den verwendeten Herstellverfahren 1.0 bis 2.5 %, bezogen auf Benzol, betragen. Unter Destillationsrückstand im Sinne der vorliegenden Erfindung sollen Stoffe verstanden werden, die bei der Synthese von Ethylbenzol anfallen und nach der Aufarbeitung des Reaktionsbemisches in der sogenannten Polyethylbenzolkolonne abgetrennt werden. In der Regel weist dieser Destillationsrückstand, bezogen auf Normaldruck, einen Siedeanfang von 200 °C und ein Siedeende von 350 °C auf. Chemisch gesehen handelt es sich hierbei um eine Vielzahl von Verbindungen, die von Spuren an Diethylbenzol über Tri- oder Tetraethylbenzol und den noch höher alkylierten Ethylbenzolen, sowie Diphenyläthanen zu hochkondensierten, basisch wirkenden Verbindungen, zum überwiegenden Teil unbekannter Struktur reichen. Bevorzugt werden für die Durchführung des erfindungsgemäßen Verfahrens etwa 2 bis 10 Gew-%, insbesondere jedoch 4 bis 8 % des Anteils, der in die PEB-Kolonne eingeführt wird, am Sumpf abgezogen und zur Aufarbeitung nach dem erfindungsgemäßen Verfahren verwendet. Dieses Sumpfprodukt (Destillationsrückstand) weist somit kein Benzol, sowie kein Ethylbenzol, allenfalls geringe Mengen an Diethylbenzol und Triethylbenzol bis maximal 15 %, sowie die höher alkylierten Benzole (Tetra-penta-hexa-ethylbenzol) in Mengen von bis zu ca. 30 Gew.-%. auf. Die höher kondensierten Verbindungen sowie die unbekannter Struktur machen den größten Teil des Rückstandes von 55 bis 70 aus. Alle Angaben sind in Gew.-%, bezogen auf den Rückstand.

Das erfindungsgemäße Verfahren kann diskontinuierlich bzw. kontinuierlich durchgeführt werden ; bevorzugt ist jedoch die letztgenannte Verfahrensweise.

Der Destillationsrückstand wird in Gegenwart von 2 bis 20 Gew.-%, insbesondere von 5 bis 10 Gew.-% an wasserfreiem AlCl$_3$, bezogen auf den Rückstand, umgesetzt. Als Cokatalysator für das Wasserfreie Aluminiumchlorid wird wasserfreier Chlorwasserstoff in vorzugsweise stöchiometrischen Mengen, bezogen auf Aluminiumchlorid, angewendet. Das erfindungsgemäße Verfahren wird bei erhöhter Temperatur durchgeführt, Temperaturen und Verweilzeiten sind in den dem Fachmann geläufigen oder leicht zu ermittelten Grenzen innerhalb der nachstehend genannten Bereiche zu variieren. Die Reaktion ist bereits bei Temperaturen wenig oberhalb Raumtemperatur bei langen Verweilzeiten durchführbar. Bevorzugt werden Temperaturen im Bereich von 30 bis 150 °C, insbesondere von 45 bis 105 °C bei Normaldruck.

Die Verweilzeit kann 20 Min. bis 6 Stunden betragen ; bevorzugte Verweilzeiten liegen bei 1,5 bis 4 Stunden.

Die Umsetzung kann in einem beliebig gestalteten Gefäß durchgeführt werden. Bevorzugt werden Rührkessel verwendet. Die Behältermaterialien sind den Reaktanten anzupassen. In der Regel wird man daher die von der Ethylbenzolsynthese geläufigen Materialien anwenden.

Die Umsetzung wird vorzugsweise unter Rühren durchgeführt. Die Rührbedingungen müssen so gewählt werden, daß eine ausreichende Durchmischung der Reaktanten gewährleistet ist. Die Bedingungen kann der Fachmann anhand der Größe des Kessels und der zur Verfügung stehenden Ruhrwerke ohne erfinderisches Zutun durch wenige Versuche festlegen. Nach der Umsetzung wird das Reaktionsprodukt in einem Trenngefäß üblicher Bauart in eine spezifisch leichtere organische Phase a) und in eine spezifisch schwerere Phase b) getrennt.

Die spezifisch leichtere organische Phase a) ist braungelb gefärbt und enthält im wesentlichen ein Aromatengemisch, daß Alkylbenzole verschiedenen Alkylierungsgrades aufweist. Der Anteil dieser Wertphase, bezogen auf den Rückstand, kann mehr als 50, insbesondere 60 bis 80 Gew.-% betragen. Der Anteil hängt von den gewählten Reaktionsbedingungen, Katalysatormengen, Verweilzeit u. dgl. ab. Es hat sich ferner als zweckmäßig erwiesen, die organische Phase a), die die Wertstoffe enthält, nicht in einer besonderen Kolonne aufzutrennen, sondern diese in der vorhandenen Äthylbenzolanlage dem Rohethylbenzol vor dessen Wäsche zuzugeben. Dies hat den Vorteil, daß bei der anschließenden Destillation noch geringe Verunreinigungen beseitigt werden können. Durch die Rückführung der spezifisch leichteren Phase a) in den Prozeß der Aufarbeitung des Reaktionsproduktes der Ethylbenzolsynthese gelingt es, mehr als 50 % des bisher anfallenden Rückstandes der Ethylbenzolsynthese der Verwertung zuzuführen. Das erfindungsgemäße Verfahren erlaubt daher die Verbesserung der Ausbeute bei den bekannten Ethylbenzolverfahren von bisher 97,5 bis 98,5 % auf ca. 98,5 bzw. bis zu ca. 99,5 %, bezogen auf eingesetztes Benzol. Dies ergibt sich bei einem Anfall von einem Destillationsrückstand in einer Menge von 1,0 bis 2,5 %, bezogen auf Ethylbenzol.

Die spezifisch schwerere Phase b) enthält im wesentlichen noch hochsiedende Aromaten und auch Katalysatorrückstände. Diese spezifisch schwerere Phase b) sollte zweckmäßigerweise zur Zersetzung noch vorhandener Aluminiumchlorid-Organyl-Komplexe mit Natronlauge und/oder Wasser behandelt werden.

Eine andere Art der Aufarbeitung des Reaktionsproduktes besteht darin, daß man das gesamte beim erfindungsgemäßen Verfahren anfallende Reaktionsgemisch, d.h. Wert- und Komplexphase in einer wäßrigen Natriumhydroxidlösung zersetzt. Die Natriumhydroxidmenge kann zwischen 3 bis 7 Mol NaOH, bezogen auf 1 Mol angewendetes Aluminiumchlorid betragen. Vorzugsweise wendet man 5 bis 6 Mol NaOH an. Das in Natronlauge umgesetzte Reaktionsprodukt wird in ein Trenngefäß übergeführt und in diesem wird die aluminiumchloridfreie organische Phase abgeschieden. Die organische Phase wird anschließend einer Distillation unter vermindertem Druck unterworfen. Die so erhaltenen Wertstoffe werden in die vorhandene Ethyl-Benzol-Kolonne der Anlage zurückgeführt. Diese Arbeitsweise hat gegenüber der vorstehend geschilderten den Vorteil, daß Wertstoffe, die noch im Aluminium-Organylkomplex enthalten sind, ebenfalls zurückgewonnen werden können.

Es ist zweckmäßig, die nach dieser Behandlung abgetrennte aromatenreiche Phase, die den gesamten nicht mehr verwertbaren Rückstand darstellt, zu filtrieren, um sie ohne Schwierigkeiten anschließend der Verbrennung, z.B. in der Diphenylanlage, zuzuführen.

Nachstehend wird, ohne das erfindungsgemäße Verfahren zu limitieren, eine mögliche Ausführungsform anhand eines Verfahrensschemas (vgl. Abbildung) erläutert. In der PEB-Kolonne (1) wird am Sumpf durch die Leitung (3) über die Pumpe (4) der Destillationsrückstand (Sumpfprodukt) inklusiv dem Cokatalysator HCl in einen als Rührbehälter ausgebildeten Reaktionsraum (10) eingeführt. Über die Vorrichtung (11) wird wasserfreies Aluminiumchlorid durch Leitung (12) zudosiert. Der Reaktionsbehälter besitzt die erforderlichen Rühr- bzw. Heiz- oder Kühleinrichtungen. Das Umsetzungsprodukt gelangt über Leitung (13) in einen Trennbehälter (14), in dem die Trennung in die Phasen a) und b) erfolgt. Die spezifisch leichtere Phase a) gelangt über Leitung (15) in den Destillationstrakt der Ethylbenzolanlage. Die spezifisch schwerere Phase b) wird aus dem Trenngefäß über die Pumpe (16) zusammen mit Wasser und Natronlauge aus den Leitungen (17) und (18) über ein Mischaggregat (19) in den Behälter (20) gegeben. In diesem Behälter, der mit einem Rührorgan ausgestattet ist, wird die Zersetzung der in der spezifisch schweren Phase b) enthaltenen Aluminiumchlorid-Organyl-Komplex vorgenommen. Der Austrag aus diesem Zersetzungsbehälter gelangt über Leitung (21) in einen ersten Trennbehälter (22), in dem bereits eine Trennung von organischer Phase und wäßriger, Lauge enthaltender Phase erfolgt. Die organische Phase wird über Leitung (23) und Pumpe (24) in ein weiteres Trenngefäß (25) eingeführt. Zusammen mit dieser organischen Phase wird durch Leitung (26) Kondenswasser zugegeben. Im Trenngefäß (25) wird der Rückstand abgetrennt und kann, ggf. zur Verbrennung bzw. Energieerzeugung bzw. zur Aufheizung des Wärmeträgers verwendet werden. Die in den Trenngefäßen abgeführten wäßrigen Phasen gelangen entweder vor oder nach Neutralisierung in das Abwasser bzw. zur endgültigen Klärung, z.B. in eine biologische Kläranlage.

Die Erfindung wird nachstehend anhand der Beispiele näher erläutert. Die in den Beispielen genannten Prozente, beziehen sich, wenn nichts anderes vermerkt ist, auf das Gewicht.

Beispiele 1 bis 12

Die nachstehend beschriebenen Versuche wurden unter den in der Tabelle genannten Bedingungen durchgeführt. Zur Durchführung der Versuche wurde eine Glasapparatur verwendet, die die in der Abbildung dargestellten Bestandteile aufwies. Zum Einsatz gelangten stündlich 1 000 Gramm eines bei der Herstellung von Ethylbenzol anfallenden Rückstandes der einen Siedebeginn von 200 °C und ein Siedeende 350 °C bei Normaldruck aufwies. Bei dem in den Beispielen verwendeten Einsatzprodukt handelte es sich um ein benzol- und ethylbenzolfreies Produkt, das ungefähr 1 % Butylbenzole, ca. 10 % Diethylbenzol, ca. 30 % Triethylbenzol und höhersiedende Polyethylbenzole sowie ungefähr 15 % 1,1-Diphenylethan und ca. 44 % Hexaethylbenzol sowie höhersiedende Substanzen aufwies. In der Tabelle sind die verwendeten Mengen an Aluminiumchlorid, in %, bezogen auf den Rückstand, aufgeführt. Zusätzlich wurde eine zu dem verwendeten wasserfreien Aluminiumchlorid äquivalente Menge wasserfreien Chlorwasserstoffs angewendet. Die Ergebnisse sind in der Tabelle ebenfalls aufgeführt. Die Wertphase ist in Prozent, bezogen auf den Rückstand, angegeben. Der nach der erfindungsgemäßen Behandlung erhaltene Rückstand ist ebenfalls, in Prozent, bezogen auf den eingesetzten Rückstand vor Durchführung des erfindungsgemäßen Verfahrens, in der Tabelle aufgeführt. Er ergibt sich aus der Differenz des erstgenannten Wertes zu 100. Die Verweilzeiten in dem als Rührbehälter ausgestatteten Reaktionsraum betrugen zwischen 20 und 360 Minuten. Der Reaktionsbehälter hatte ein Fassungsvermögen von 4 Litern, bei einem Durchmesser von ca. 170 mm und einer Höhe von ca. 180 cm. Es handelt sich um einen Glaskolben, der mit einem Glas-Teflon-Intensivrührer mit beweglichem Teflonblatt und KPG-Welle ausgestattet war. Die Abmessungen des Rührerblattes betrugen 24 × 90 mm, der Wellendurchmesser betrug 10 mm. Die Rührgeschwindigkeit konnte im Bereich zwischen 0 und 1 000 Umdrehungen pro Minute eingestellt werden. Bei der Durchführung der Beispiele wurden Drehzahlen im Bereich von 300 bis 600 Umdrehungen pro Minute bei dem vorstehend genannten Dimensionen des Rühres und des Behälters gewählt.

Es wurde dabei in allen Fällen eine für das erfindungsgemäße Verfahren ausreichende Homogenisierung des Reaktionsmediums beobachtet. In dem dem Reaktionsbehälter nachgeschalteten Trennbehälter wurde die spezifisch leichtere Wertphase a) abgetrennt. Der Gewichtsanteil dieser Wertphase, bezogen auf das eingesetzte Produkt, ist in der Tabelle für die verschiedenen Versuche aufgeführt. Die im Trennbehälter anfallende spezifisch schwerere Phase b) wurde nach der Abtrennung mit verdünnter Natronlauge zersetzt. Die dabei entstehende nicht mehr verwertbare organische Phase ist in der Tabelle als Rückstand, jeweils bezogen auf den Einsatz, angegeben. Sowohl die Wertphase a) als auch die spezifisch schwerere Phase b) wurden im vorliegenden Fall verworfen und nicht, wie dies in technischen Anlagen bevorzugt wird, der weiteren Verwertung zugeführt.

Es hat sich herausgestellt, daß bei der Änderung der Verweilzeit in etwa dieselben Mengen an Wertphase erhalten werden können, wenn man Verweilzeiten von 20 Minuten oder höhere Verweilzeiten von bis zu 360 Minuten anwendet, sofern die Temperatur in dem genannten Bereich nicht zu hoch gewählt wird.

(Siehe die Tabelle, Seite 5)

# 0 036 130

TABELLE

| Beispiel | % AlCl$_3$ | Temperatur °C | Verweilzeit min | Wertphase | Rückstand |
|---|---|---|---|---|---|
| Rückstand | — | — | — | — | 100 |
| 1 | 2 | 50° | 120 | 90,5 | 9,5 |
| 2 | 4 | 50° | 120 | 82,0 | 18,0 |
| 3 | 6 | 50° | 120 | 72,7 | 27,3 |
| 4 | 8 | 50° | 120 | 62,0 | 38,0 |
| 5 | 10 | 50° | 120 | 53,3 | 46,7 |
| 6 | 20 | 50° | 120 | 48,0 | 52,0 |
| 7 | 2 | 100° | 120 | 88,8 | 11,2 |
| 8 | 4 | 100° | 120 | 78,8 | 21,2 |
| 9 | 6 | 100° | 120 | 71,2 | 28,8 |
| 10 | 8 | 100° | 120 | 56,9 | 43,1 |
| 11 | 10 | 100° | 120 | 53,8 | 46,2 |
| 12 | 20 | 100° | 120 | 48,0 | 52,0 |

## Ansprüche

1. Verfahren zur Umsetzung des bei der Synthese von Ethylbenzol durch Alkylieren von Benzol mit Ethylen in Gegenwart von Katalysatoren erhaltenen und anschließend abgetrennten Destillationsrückstandes, wobei man den Destillationsrückstand in Abwesenheit von Benzol in Gegenwart von 2 bis 20 Gew.-% wasserfreiem Aluminiumchlorid, bezogen auf den Rückstand und in Gegenwart von etwa äquimolaren Mengen Chlorwasserstoff, bezogen auf Aluminiumchlorid, bei Temperaturen von 30 bis 150 °C und bei Verweilzeiten von 20 Minuten bis zu 6 Stunden, ggf. unter Rührern umsetzt und das Reaktionsprodukt in eine

a) spezifisch leichtere organische Phase, die im wesentlichen die Wertstoffe enthält, und

b) eine spezifisch schwerere Phase, die im wesentlichen hochsiedende Aromaten und Katalysatorrückstände enthält, trennt

und aus der spezifisch leichteren organischen Phase a) die Wertstoffe in üblicher Weise durch Destillation gewinnt.

2. Verfahren nach Anspruch 1, dadurch gekennzeichnet, daß die spezifisch schwerere Phase b) mindestens in einer weiteren Verfahrensstufe mit Wasser und/oder Natronlauge behandelt wird.

## Claims

1. A process for the reaction of the distillation residue which is formed in the synthesis of ethylbenzene by alkylating benzene with ethylene in the presence of catalysts, and subsequently separated off, wherein the distillation residue is reacted in the absence of benzene in the presence of from 2 to 20 % by weight, based on the residue, of anhydrous aluminium chloride and in the presence of approximately equimolar amounts of hydrogen chloride, based on aluminium chloride, at a temperature of from 30° to 150 °C, the residence time being from 20 minutes to 6 hours, with or without stirring, and the reaction product is separated into

a) an organic phase of low specific gravity which consists essentially of the products of value, and

b) a phase of high specific gravity which consists essentially of high-boiling aromatics and catalyst residues,

and the products of value are recovered from the organic phase of low specific gravity a) in a conventional manner by distillation.

2. A process as claimed in claim 1, wherein the phase of high specific gravity b) is treated in at least one further procedural step with water and/or aqueous caustic solution.

## Revendications

1. Procédé pour la conversion du résidu de la distillation formé lors de la synthèse de l'éthylbenzène par alcoylation du benzène par l'éthylène en présence de catalyseurs et séparé après la réaction, dans lequel on fait réagir le résidu de distillation, en l'agitant le cas échéant, pendant des durées de 20 minutes à 6 heures à des températures de 30 à 150 °C en présence de 2 à 20 % en poids par rapport au résidu de chlorure d'aluminium anhydre et en présence d'une proportion approximativement équimolaire

5

**0 036 130**

d'hydrogène chloré par rapport au chlorure d'aluminium, mais en l'absence de benzène, puis on divise le produit réactionnel obtenu en .

a) une phase organique spécifiquement plus légère, qui contient essentiellement les produits recherchés et

b) une phase spécifiquement plus lourde, qui contient essentiellement les substances aromatiques à point d'ébullition plus élevé et les résidus du catalyseur,

les produits recherchés étant ensuite extraits de manière usuelle de la phase organique spécifiquement plus légère a) par une distillation.

2. Procédé suivant la revendication 1, caractérisé en ce que la phase spécifiquement plus lourde b) est traitée dans au moins un stade opératoire supplémentaire par de l'eau et (ou) une solution d'hydroxyde de sodium.

6